# EUROPÄISCHE PATENTSCHRIFT

(11) **EP 0 805 169 B1**
(45) Veröffentlichungstag und Bekanntmachung des Hinweises auf die Patenterteilung: **18.06.2003**
(21) Anmeldenummer: 97107231.9
(22) Anmeldetag: 30.04.1997
(51) Int. Cl.: C08F 20/12, C08F 2/22, C08F 6/14, C08F 291/00, A61K 7/06

(54) **Verfahren zur Herstellung von im alkalischen löslichen Copolymerisaten auf (Meth) Acrylatbasis**
Process for preparing in-basic-medium soluble copolymers based on (meth)acrylates
Procédé de préparation de copolymères à base de (méth)acrylates solubles en milieu alcalin

(30) Priorität: 02.05.1996 DE 19617633
(43) Veröffentlichungstag der Anmeldung: 05.11.1997
(73) Patentinhaber: BASF AKTIENGESELLSCHAFT, 67056 Ludwigshafen (DE)
(72) Erfinder: Schehlmann, Volker, Dr., 67354 Römerberg (DE)
(74) Vertreter: Kinzebach, Werner, Dr.

(56) Entgegenhaltungen:
- EP-A- 0 501 272
- EP-A- 0 646 606
- DE-A- 4 314 305
- FR-A- 1 343 230

## Beschreibung

Die vorliegende Erfindung betrifft ein Verfahren zur Herstellung von im alkalischen löslichen Copolymerisaten auf (Meth)acrylatbasis durch radikalische Emulsionspolymerisation bei einem pH <4 sowie die nach diesem Verfahren erhältlichen Copolymerisate und deren Verwendung.

Copolymerisate auf (Meth)acrylatbasis, die im alkalischen wasserlöslich sind, werden häufig auf dem Gebiet der Kosmetik als Haarfestiger eingesetzt. So beschreibt beispielsweise die EP-A-379 082 Haarfestigungsmittel, welche als Filmbildner Copolymerisate mit einem K-Wert von 10 bis 50 enthalten, die aufgebaut sind aus 75 bis 99 Gew.-% t-Butylacrylat und/oder t-Butylmethacrylat, 1 bis 25 Gew.-% Acrylsäure und/oder Methacrylsäure und 0 bis 10 Gew.-% eines weiteren radikalisch copolymerisierbaren Monomeren. Zur Anwendung in der Kosmetik werden die Carboxylgruppen der Copolymerisate teilweise oder vollständig durch Amine neutralisiert.

Die DE-A-43 14 305 beschreibt Haarfestigungsmittel, welche als Filmbildner Copolymerisate der in der EP-A-379 082 genannten Art enthalten, die aufgebaut sind aus 30 bis 72 Gew.-% t-Butylacrylat und/oder t-Butylmethacrylat, 10 bis 28 Gew.-% Acrylsäure und/oder Methacrylsäure und 0 bis 60 Gew.-% eines radikalisch copolymerisierbaren Monomeren oder einer radikalisch copolymerisierbaren monomeren Mischung, wobei mindestens eines dieser Monomeren ein Homopolymerisat mit einer Glastemperatur <30 °C liefert.

Die EP-A-646 606 beschreibt alkalilösliche Copolymerisate mit niedrigem Molekulargewicht, die aufgebaut sind aus 25 bis 75 Gew.-% eines wasserunlöslichen monoethylenisch ungesättigten, aromatischen Monomers, und 25 bis 75 Gew.-% (Meth)acrylsäure. Copolymerisate dieser Art finden Anwendung in Druckpasten, Oberflächenbehandlungsmitteln, Anstrichmitteln, Klebstoffzusammensetzungen und Papierbeschichtungsmitteln. Diese Copolymerisate werden durch Emulsionspolymerisation bei einem pH ≤4,5 unter Verwendung eines Molekulargewichtreglers hergestellt.

Wenn die erwähnten Copolymerisate als Haarfestiger Anwendung finden, muß gewährleistet sein, daß sie gut vom Haar auswaschbar sind. Im allgemeinen wird dies erreicht, indem man die Copolymerisate durch teilweise oder vollständige Neutralisation der Carboxylgruppen wasserlöslich macht. Zur Neutralisation der Carboxylgruppen verwendet man im allgemeinen ein Alkalimetallhydroxid oder, vorzugsweise, ein organisches Amin. Der pH-Wert einer Lösung eines teilweise oder vollständig neutralisierten Copolymerisates liegt im allgemeinen im Bereich von 8,0 bis 9,5.

Die Herstellung der Copolymerisate kann durch Lösungs-, Suspensions- oder Emulsionspolymerisation erfolgen. Dabei hat die Emulsionspolymerisation den Vorteil, daß die erhaltene Dispersion durch physikalische Desodorierung, beispielsweise durch Einleiten von Wasserdampf, von Restmonomeren und Geruchsträgern befreit werden kann.

Die durch Emulsionspolymerisation erhaltenen Dispersionen zeigen jedoch die beiden folgenden Nachteile:
a) Sie besitzen keine ausreichende Scherstabilität. Die üblicherweise zur physikalischen Desodorierung herangezogenen technischen Vorrichtungen führen zu einer starken Scherung der Dispersion. Bei nicht hinreichend scherstabilen Dispersionen hat dies zur Folge, daß teilweise oder vollständige Koagulation und/oder Sedimentation zu beobachten ist und/oder Beläge in den Apparaturen gebildet werden.
b) Die nach der Polymerisation erhaltenen Dispersionen sind aufgrund der Anwesenheit der Carboxylgruppen sauer und zeigen bei 6-monatigen Lagertests eine pH-Erniedrigung um ca. 0,3 Einheiten. Dies ist in der Praxis unerwünscht und nicht akzeptabel.

Der vorliegenden Erfindung liegt daher die Aufgabe zugrunde, ein Verfahren zur Herstellung von im alkalischen löslichen Copolymerisaten auf (Meth)acrylatbasis durch radikalische Emulsionspolymerisation zu schaffen, das eine Copolymerisatdispersion mit ausreichender Stabilität liefert. Insbesondere soll die Copolymerisatdispersion eine bessere Scherstabilität und Lagerstabilität aufweisen als die Dispersionen des Standes der Technik.

Überraschenderweise wurde nun gefunden, daß diese Aufgabe gelöst wird, wenn man den pH-Wert der nach der Polymerisation erhaltenen Dispersion auf einen Wert im Bereich von 4 bis 7 einstellt.

Gegenstand der vorliegenden Erfindung ist daher ein Verfahren zur Herstellung von im alkalischen löslichen Copolymerisaten auf (Meth)acrylatbasis durch radikalische Emulsionspolymerisation bei einem pH <4, das dadurch gekennzeichnet ist, daß man den pH-Wert der Dispersion nach der Polymerisation durch Zugabe einer Base auf einen Wert im Bereich von 4 bis 7 einstellt.

Die Copolymerisate werden erhalten durch radikalische Polymerisation von
a) 30 bis 72 Gew.-% t-Butylacrylat oder t-Butylmethacrylat oder eines Gemisches davon,
b) 10 bis 28 Gew.-% Acrylsäure oder Methacrylsäure oder eines Gemisches davon und
c) 0 bis 60 Gew.-% mindestens eines weiteren radikalische copolymerisierbaren Monomeren, wobei bevorzugt wenigstens eines dieser Monomere ein Homopolymerisat mit einer Glastemperatur ≤ 30°C liefert.

Zur Modifikation der Eigenschaften des Copolymerisates kann noch wenigstens ein weiteres Monomer einpolymerisiert sein. Dieses Monomer oder mindestens eines dieser Monomere soll dabei ein Homopolymerisat mit einer Glastemperatur ≤30 °C liefern. Vorzugsweise handelt es sich dabei um C₁-C₁₈-Alkylacrylate oder C₁-C₁₈-Alkyl(meth)acrylate oder N-C₁-C₁₈-Alkylacrylamide oder N-C₁-C₁₈-Alkyl(meth)acrylamide, insbesondere N-C₁-C₄-Alkylacrylamide oder -Methacrylamide oder Gemische von zwei oder mehreren dieser Monomere. Als C₁-C₄-Alkylreste in den genannten (Meth)acrylaten und (Meth)acrylamiden kommen Methyl, Ethyl, n-Propyl, isoPropyl, n-Butyl, iso-Butyl, sec-Butyl ur.d t-Butyl in Betracht. Besonders bevorzugte Monomere sind Ethylacrylat oder ein Gemisch aus Ethylacrylat und N-t-Butylacrylamid.

Vorzugsweise verwendet man 50 bis 72 Gew.-% und insbesondere 60 bis 70 Gew.-% des Monomeren a),
10 bis 25 Gew.-% und insbesondere 15 bis 23 Gew.-% des Monomeren b) und
3 bis 38 Gew.-% und insbesondere 7 bis 25 Gew.-% des Monomeren c) .

Die besonders bevorzugten Copolymerisate sind in der DE-A-43 14 305 beschrieben, auf deren Inhalt hiermit in vollem Umfang Bezug genommen wird.

Die K-Werte der erfindungsgemäßen Polymerisate liegen im allgemeinen im Bereich von 10 bis 90, vorzugsweise 10 bis 60 und insbesondere 15 bis 50. Der gewünschte K-Wert läßt sich in üblicher Weise durch Wahl der Polymerisationsbedingungen, beispielsweise der Polymerisationstemperatur und der Initiatorkonzentration einstellen. Der K-Wert kann auch durch die Anwendung von Reglern, wie Aldehyden, Halogenverbindungen oder Schwefelverbindungen, beispielsweise Formaldehyd, Acetaldehyd, Bromtrichlormethan, Mercaptoethanol, 2-Ethylhexylthioglycolat, Thioglykolsäure oder Dodecylmercaptan, reduziert werden. Die Mengen an Reglern, bezogen auf die zu polmerisierenden Polymeren, betragen im allgemeinen bis zu 10 Gew.-%, vorzugsweise 0,1 bis 5 Gew.-%.

Die K-Werte werden nach Fikentscher, Cellulosechemie, Band 13, Seiten 58 bis 64 (1932) bei 25°C in 1 gew-%iger ethanolischer Lösung gemessen und stellen ein Maß für das Molekulargewicht dar.

Die Glastemperatur der erfindungsgemäßen Copolymerisate liegt üblicherweise im Bereich von 50 bis 130°C, insbesondere 60 bis 100°C.

Die Herstellung der Copolymerisate erfolgt in üblicher Weise unter Verwendung von Initiatoren, wie Peroxo- oder Azoverbindungen, beispielsweise Dibenzoylperoxid, t-Butylperpivalat, t-Butylper-2-ethylhexanoat, Di-t-Butylperoxid, t-Butylhydroperoxid, 2,5-Dimethyl-2,5-di(t)butylperoxy(hexan), Alkalimetall- oder Ammoniumpersulfate, Azo-bis-isobutyronitril, 2,2'-Azo-bis-(2-Methylbutyronitril), 2,2'-Azo-bis-(2,4-dimethylvaleronitril), 1,1'-Azo-bis-(1-cyclohexancarbonitril), 2,2'-Azobis (2-amidinopropan)salze, 4,4'-Azobis(4-cyanovaleriansäure) oder 2-(Carbamoylazo)-isobutyronitril etc., Wasserstoffperoxid oder Redoxinitiatoren. Die Initiatoren werden üblicherweise in Mengen bis zu 10, vorzugsweise 0,02 bis 5 Gew.-%, bezogen auf die zu polymerisierenden Monomeren eingesetzt.

Die Polymerisation wird in Anwesenheit eines für diese Zwecke üblichen Emulgators und/oder Schutzkolloids durchgeführt. Geeignete Schutzkolloide sind beispielsweise Polyvinylalkohole, Cellulosederivate oder Polyvinylpyrrolidone. Die Emulgatoren können anionischer, kationischer oder nicht-ionischer Natur sein. Geeignete Emulgatoren sind beispielsweise ethoxylierte Mono-, Di- und Trialkylphenole, ethoxylierte Fettalkohole oder Sorbitanester, Alkali- und Ammoniumsalze von Alkylsulfaten oder Alkylethersulfaten, von Alkylsulfonsäuren, von Ligninsulfonsäure und von Alkylarylsulfonsäuren oder Alkyldiphenyloxidsulfonate.

Die Emulsionspolymerisation erfolgt üblicherweise unter Sauerstoffausschluß bei Temperaturen im Bereich von 20 bis 200°C. Die Polymerisation kann diskontinuierlich oder kontinuierlich durchgeführt werden.

Die Mengen an Monomeren und Dispergiermittel wählt man zweckmäßigerweise so, daß man eine 30 bis 80 gew.-%ige Dispersion der Copolymerisate enthält.

Vorzugsweise dosiert man zumindest einen Teil der Monomere, Initiatoren und gegebenenfalls Regler während der Polymerisation gleichmäßig in das Reaktionsgefäß. Die Monomere und der Initiator können jedoch auch im Reaktor vorgelegt und polymerisiert werden, wobei gegebenenfalls gekühlt werden muß.

Gemäß einer bevorzugten Ausführungsform führt man die Polymerisation unter Verwendung eines Saatlatex durch. Zweckmäßigerweise wird der Saatlatex aus den zu polymerisierenden Polymeren in der ersten Polymerisationsphase in üblicher Weise hergestellt. Anschließend gibt man den verbleibenden Teil der Monomerenmischung, vorzugsweise nach dem Zulaufverfahren, zu.

Nach beendeter Polymerisation kann man zur Senkung des Restpolymerengehaltes eine Nachpolymerisation unter Zugabe geeigneter Initiatoren in bekannter Weise durchführen. Gewünschtenfalls kann auch eine physikalische Desodorierung in üblicher Weise erfolgen, beispielsweise durch Einleiten von Wasserdampf.

Die Einstellung des pH-Wertes der Dispersion auf 4 bis 7, vorzugsweise 5 bis 7 und insbesondere 5,5 bis 7, erfolgt durch Zugabe üblicher Basen, z.B. ein Alkalimetallhydroxid, wie NaOH, Ammoniak oder organische Amine, vorzugsweise solche, die auch zur weiteren Neutralisation der Carboxylgruppen verwendet werden, um das Copolymerisat leicht auswaschbar zu machen. Geeignete Amine sind beispielsweise Mono-, Di- oder Trialkanolamine, wie Mono-, Di- oder Triethanolamin, Triisopropanolamin oder 2-Amino-2-methylpropanol, Alkandiolamine, wie 2-Amino-2-ethylpropan-1,3-diol, oder primäre, sekundäre oder tertiäre Alkylamine, wie N-N-Diethylpropylamin.

Die Einstellung des pH-Wertes auf 4 bis 7 kann gegebenenfalls auch durch die Zugabe einer Pufferlösung erfolgen. Bevorzugte Puffer basieren auf Alkali- bzw. Ammoniumcarbonat oder -bicarbonat.

Die Basen bzw. Puffer werden vorzugsweise als verdünnte wäßrige Lösung zu der Dispersion gegeben.

Die erfindungsgemäßen Copolymerisate sind insbesondere in der Kosmetik als Filmbildner brauchbar. Sie weisen eine gute haarfestigende Wirkung auf und zeichnen sich dadurch aus, daß sie das Haar praktisch nicht verkleben.

Die nach dem erfindungsgemäßen Verfahren erhältliche Copolymerisatdispersion kann entweder direkt in eine wäßrige Haarfestigerzubereitung eingearbeitet werden oder es kann eine Trocknung vorgenommen werden, beispielsweise eine Sprühtrocknung, so daß das Copolymerisat als Pulver verwendet und in ein Haarbehandlungsmittel eingearbeitet werden kann.

Pulver, die durch Trocknung von erfindungsgemäßen Dispersionen erhalten werden, sollten für die Anwendung in haarkosmetischen Formulierungen vorzugsweise in Ethanol löslich sein.

Zur Verwendung in Haarbehandlungsmitteln werden die in dem Polymerisat enthaltenen Carboxylgruppen teilweise oder vollständig mit einem Alkalimetallhydroxid oder einem Amin neutralisiert, zweckmäßigerweise zu 30 bis 100 %, vorzugsweise zu 50 bis 100 %. Als Amine sind die oben erwähnten Amine brauchbar.

Die nachfolgenden Beispiele erläutern die Erfindung ohne sie zu begrenzen. Die nachfolgend angegebenen Testdaten wurden wie folgt erhalten:

### Scherstabilität:

Eine Probe der Dispersion wurde 15 Minuten mit einer Geschwindigkeit von 9000 Upm gerührt. Als Maß für die Scherstabilität wurde die Teilchengrößenverteilung, gemessen durch Lichtstreuung, bestimmt. Bei einer scherstabilen Dispersion ändert sich bei der hier vorgenommenen Scherbelastung die Teilchengrößenverteilung nicht signifikant. Bei einem Unterschied von mehr als 5 % ist die Dispersion gegen Scherung instabil.

### Klarlöslichkeit in Ethanol:

Eine Probe der Dispersion wird durch Gefrier- oder Sprühtrocknung in ein Pulver überführt, in Ethanol gelöst (5 % w/w) und mittels 2-Amino-2-methylpropanol zu 100 % neutralisiert.

Die Prüfung auf Klarlöslichkeit erfolgt visuell. Die Beurteilung kann sein: klar, fast klar, trüb und stark trüb. Aus anwendungstechnischer Sicht sind die Klassifizierungen klar und fast klar akzeptabel.

### Klarlöslichkeit in Wasser:

Die Prüfung erfolgt analog der Klarlöslichkeit in Ethanol. pH-Lagerstabilität:

Der pH der Dispersion nach 6-monatiger Lagerung bei 25°C wird gemessen. Die Dispersion ist lagerstabil, wenn sich der pH um maximal 0,15 pH-Einheiten ändert.

### Koagulatanteil:

Der Koagulatanteil der Dispersion bezogen auf die Monomereinwaage, wird durch Filtration bestimmt. Koagulatbildung liegt vor, wenn der Koagulatanteil ≥ 0,1 % ist.

Für die nachfolgend beschriebenen Beispiele und Vergleichsbeispiele wurde eine Dispersion wie folgt hergestellt:

Aus 1 g Natriumlaurylsulfat, 6,7 g eines handelsüblichen nichtionischen Emulgators, 100 g Wasser, 1,3 g Ethylhexylthioglycolat, 60 g Methacrylsäure, 210 g tert.-Butylacrylat und 30 g Ethylacrylat stellt man eine Emulsion her, die im Zulaufverfahren in ein Polymerisationsgefäß, das 500 g Wasser enthält, innerhalb von ca. 2 Stunden bei ca. 75 - 85°C gegebenen wurde. Gleichzeitig wurde der Initiator, 1 g Nariumpersulfat gelöst in Wasser, ebenfalls kontinuierlich zugefahren. Nach Zulaufende wird 1 - 2 h bei der angegebenen Temperatur nachpolymerisiert.

Dieses Verfahren wird gemäß den nachfolgenden Beispielen 1 bis 5 und Vergleichsbeispielen 1 bis 4 modifiziert.

### Beispiel 1

Nach beendeter Polymerisation wurden 2 g Ammoniak als wäßrige Lösung zu der Dispersion gegeben. Der pH-Wert der Lösung war 5,8.

### Beispiel 2

Nach beendeter Polymerisation wurden 2 g Ammoniak in Form einer wäßrigen Ammoniumhydrogencarbonatlösung zu der Dispersion gegeben. Der pH-Wert der Lösung war 5,8.

### Beispiel 3

Nach beendeter Polymerisation wurden 1,8 g Ammoniak in Form einer wäßrigen Ammoniumcarbonatlösung zu der Dispersion gegeben. Der pH-Wert der Lösung war 5,7.

### Beispiel 4

Nach beendeter Polymerisation wurden 8,5 g 2-Amino-2-methylpropanol als wäßrige Lösung zu der Dispersion gegeben. Der pH-Wert der Lösung war 5,9.

### Beispiel 5

Zu Beginn der Polymerisation gab man ca. 10 % der eingesetzten Monomeremulsion direkt in die Vorlage und stellte daraus mit dem entsprechenden Anteil an Initiatorlösung in der ersten Polymerisationsphase einen Saatlatex her. In der zweiten Polymerisationsphase gab man die verbleibende Monomermischung und die verbleibende Initiatorlösung voneinander getrennt kontinuierlich in die Vorlage. Nach beendeter Polymerisation stellte man den pH-Wert durch Zugabe von 2,0 g Ammoniak als wäßrige Lösung auf >5 ein.

### Vergleichsbeispiel 1

Es wurde eine Dispersion nach dem in der DE-A-4314305, Beispiel 2, beschriebenen Verfahren ohne jegliche pH-Modifikation hergestellt.

### Vergleichsbeispiel 2

Zum Emulsionszulauf wurden 2 g Ammoniak als wäßrige Lösung gegeben.

### Vergleichsbeispiel 3

In die Vorlage wurden 2 g Ammoniak als wäßrige Lösung gegeben.

### Vergleichsbeispiel 4

Zum Emulsionszulauf und in die Vorlage wurden jeweils 1 g Ammoniak als wäßrige Lösung gegeben.

Mit den nach den Beispielen und Vergleichsbeispielen erhaltenen Dispersionen wurden die oben beschriebenen Tests durchgeführt. Die erhaltenen Ergebnisse sind in der Tabelle zusammengestellt:

**Tabelle**

| Beispiel Nr. | Scherstabilität | Löslichkeit in Ethanol | Löslichkeit in Wasser | Δ pH | Koagulat |
|---|---|---|---|---|---|
| 1 | stabil | fast klar | klar | < 0,2 | < 0,1% |
| 2 | stabil | fast klar | klar | < 0,2 | < 0,1% |
| 3 | stabil | fast klar | klar | < 0,2 | < 0,1% |
| 4 | stabil | fast klar | klar | < 0,2 | < 0,1% |
| 5 | stabil | klar | klar | < 0,2 | < 0,1% |

| Vergl. beisp. | | | | | |
|---|---|---|---|---|---|
| 1 | instabil | fast klar | klar | 0,3 | < 0,1% |
| 2 | stabil | stark trüb | stark trüb | < 0,2 | > 3% |
| 3 | stabil | stark trüb | trüb | < 0,2 | > 3% |
| 4 | stabil | stark trüb | stark trüb | < 0,2 | > 3% |

Das Vergleichsbeispiel 1 repräsentiert das im Stand der Technik (DE-A-43 14 305 und EP-A-646 606) beschriebene Verfahren. Es ist ersichtlich, daß diese Dispersion zwar die Anforderungen an die Klarlöslichkeit und die Bildung von Koagulat erfüllen, ihre Scherstabilität und pH-Stabilität aber ungenügend ist.

Die Vergleichsbeispiele 2 bis 4 zeigen, daß durch Erhöhung des pH-Wertes während der Polymerisation zwar die gewünschte Scherstabilität und pH-Stabilität erreicht werden kann, dagegen aber die Löslichkeit der Copolymerisate und die Bildung von Koagulat nicht mehr den Anforderungen entspricht.

Im Gegensatz dazu sind die nach dem erfindungsgemäßen Verfahren erhaltenen Dispersionen scherstabil und pH-stabil, in Wasser und Ethanol-ausreichend löslich und es ist keine Koagulatbildung zu beobachten. Es werden also einander entgegenstehende Anforderungen gleichzeitig erfüllt.

## Patentansprüche

1. Verfahren zur Herstellung von im alkalischen löslichen Copolymerisaten durch radikalische Emulsionspolymerisation bei einem pH <4 von einem Gemisch aus
a) 30 bis 72 Gew.-% t-Butylacrylat oder t-Butylmethacrylat oder einem Gemisch davon,
b) 10 bis 28 Gew.-% Acrylsäure oder Methacrylsäure oder einem Gemisch davon und
c) 0 bis 60 Gew.-% mindestens eines weiteren radikalisch copolymerisierbaren Monomeren,
**dadurch gekennzeichnet, daß** man den pH der Dispersion nach der Polymerisation durch Zugabe einer Base auf einen Wert im Bereich von 4 bis 7 einstellt.

2. Verfahren nach Anspruch 1, **dadurch gekennzeichnet, daß** man den pH der Dispersion auf einen Wert im Bereich von 5 bis 7 und insbesondere 5,5 bis 7 einstellt.

3. Verfahren nach einem der vorhergehenden Ansprüche, **dadurch gekennzeichnet, daß** wenigstens eines der Monomere (c) ein Homopolymerisat mit einer Glastemperatur <30°C liefert.

4. Verfahren nach einem der vorhergehenden Ansprüche, **dadurch gekennzeichnet, daß** man als Monomere (c) Ethylacrylat oder ein Gemisch aus Ethylacrylat und N-t-Butylacrylamid verwendet.

5. Verfahren nach einem der vorhergehenden Ansprüche, **dadurch gekennzeichnet, daß** man die Emulsionspolymerisation unter Verwendung eines Saatlatex aus den zu polymerisierenden Monomeren durchführt.

6. Copolymerisatdispersion, erhältlich nach einem Verfahren aus einem der Ansprüche 1 bis 5.

7. Verwendung der Copolymerisatdispersion nach Anspruch 6 in Haarbehandlungsmitteln.

## Claims

1. A process for preparing alkali-soluble copolymers by free-radical emulsion polymerization at a pH <4 from a mixture of
a) from 30 to 72 % by weight of t-butyl acrylate or t-butyl methacrylate or a mixture thereof,
b) from 10 to 28 % by weight of acrylic acid or methacrylic acid or a mixture thereof, and
c) from 0 to 60 % by weight of at least one further free-radically copolymerizable monomer,
which comprises adjusting the pH of the dispersion after polymerization to a level within the range from 4 to 7 by adding a base.

2. A process as claimed in claim 1, wherein the pH of the dispersion is adjusted to a value in the range from 5 to 7 and in particular from 5.5 to 7.

3. A process as claimed in either of the preceding claims, wherein at least one of the monomers (c) produces a homopolymer having a glass transition temperature <30°C.

4. A process as claimed in any of the preceding claims, wherein the monomers (c) used comprise ethyl acrylate or a mixture of ethyl acrylate and N-t-butylacrylamide.

5. A process as claimed in any of the preceding claims, wherein the emulsion polymerization is carried out using a seed latex of the monomers to be polymerized.

6. A copolymer dispersion obtainable by a process as claimed in any of claims 1 to 5.

7. The use of a copolymer dispersion as claimed in claim 6 in hair treatment compositions.

## Revendications

1. Procédé pour la préparation de copolymères solubles en milieu alcalin par polymérisation radicalaire en émulsion à un pH inférieur à 4 d'un mélange de
a) 30 à 72 % en poids d'acrylate de tert-butyle ou de méthacrylate de tert-butyle ou d'un mélange de ces monomères,
b) 10 à 28 % en poids d'acide acrylique ou d'acide méthacrylique ou d'un mélange de ces monomères et
c) 0 à 60 % en poids d'au moins un autre monomère apte à la copolymérisation radicalaire,
**caractérisé par le fait que**, après la polymérisation, on règle le pH de la dispersion à l'aide d'une base à une valeur se situant dans l'intervalle de 4 à 7.

2. Procédé selon la revendication 1, **caractérisé par le fait que** l'on règle le pH de la dispersion à une valeur dans l'intervalle de 5 à 7, plus spécialement de 5,5 à 7.

3. Procédé selon l'une des revendications qui précèdent, **caractérisé par le fait que** l'un au moins des monomères (c) donne un homopolymère dont la température de transition du second ordre est inférieure à 30°C.

4. Procédé selon l'une des revendications qui précèdent, **caractérisé par le fait que** l'on utilise en tant que monomère (c) l'acrylate d'éthyle ou un mélange d'acrylate d'éthyle et de N-tert-butylacrylamide.

5. Procédé selon l'une des revendications qui précèdent, **caractérisé par le fait que** l'on procède à la polymérisation en émulsion avec utilisation d'un latex d'ensemencement des monomères à polymériser.

6. Dispersion de copolymère obtenue par un procédé selon l'une des revendications 1 à 5.

7. Utilisation de la dispersion de copolymère selon la revendication 6 en tant que produit pour le traitement de la chevelure.
